# EUROPEAN PATENT SPECIFICATION

(11) **EP 0 546 034 B1**
(45) Date of publication and mention of the grant of the patent: **17.09.1997**
(21) Application number: 91915737.0
(22) Date of filing: 09.08.1991
(51) Int. Cl.: C07C 5/27

(54) **PARAFFIN ISOMERIZATION**
ISOMERISIERUNG VON PARAFFIN
ISOMERISATION DE PARAFFINE

(30) Priority: 23.08.1990 US 571387
(43) Date of publication of application: 16.06.1993
(73) Proprietor: MOBIL OIL CORPORATION (a New York corporation), Fairfax, Virginia 22037 (US)
(72) Inventor: DEL ROSSI, Kenneth, Joseph, Woodbury, NJ 08096 (US); HUSS, Albin, Jr., Chadds Ford, PA 19317 (US); KIRKER, Garry, Wayne, Sewell, NJ 08080 (US)
(74) Representative: Kador & Partner
(86) International application number: US9105680
(87) International publication number: WO9203396

(56) References cited:
- US-A- 4 734 539
- US-A- 4 855 530
- US-A- 4 919 789
- US-A- 4 962 250
- US-A- 4 990 710
- US-A- 5 037 529
- US-A- 5 082 984

## Description

This invention relates to paraffin isomerization.

Paraffin isomerization is frequently encountered in petroleum refining and is used to convert linear (straight chain) paraffins to branched chain paraffins. In such a process, as conventionally operated, low molecular weight C₄ - C₆ paraffins are converted to iso-paraffins in the presence of an acidic catalyst such as aluminum chloride. Recently, C₆+, preferably C₁₀+ n-paraffins, have been isomerized, in the presence of large pore size zeolites to produce branched chain paraffins by skeletal rearrangement. The latter process can find application in dewaxing.

Isomerization is one of several reactions which occur in reforming of naphthas. Reforming of naphthas is undertaken to upgrade a low octane naphtha to a higher octane effluent. One of the octane enhancing reactions which occurs during reforming is the isomerization of n-paraffins to isoparaffins. Under the process conditions of reforming, other reactions which occur are aromatization (or dehydrocyclization), dehydrogenation, with some cracking.

Paraffin isomerization catalysts may also be employed as ring opening catalysts for removal of cyclic aromatic precursors from reformer feedstocks. For example, cyclohexane, a precursor to benzene, is rearranged over commercial paraffin isomerization catalysts to a mixture of branched paraffins. Branched paraffins are only partly aromatized in reforming whereas cyclohexane is completely converted to aromatics, mostly benzene. Application of paraffin isomerization catalysts for ring opening aromatics precursors will no doubt become more important as environmental regulations limiting aromatics in gasoline become more stringent.

According to the present invention there is provided a process for isomerizing a paraffin having 4 to 10 carbon atoms, said process being defined in claim 1.

The catalytic isomerization process of the invention is effective to increase the octane of low octane naphthas containing C₆-C₁₀ n-paraffins and/or mono-methyl branched paraffins, which under conventional reforming conditions, are the most difficult components to upgrade. A potential advantage of the process of the invention is an increase in liquid yields by minimizing the cracking of C₅+ hydrocarbons which include C₆ to C₁₀ n-paraffins. Still another application of the isomerization process of the invention is to upgrade refinery streams rich in C₄-C₆ n-paraffins. The potential significance of the process of the invention is readily apparent from a review of the following table of Octane Numbers of Pure Hydrocarbons from "Catalysis," Vol. VI, P.H. Emmett (ed.) Copyright 1958 by Litton Educational Publishing Company:

| Octane Numbers of Pure Hydrocarbons | |
|---|---|
| Hydrocarbon | Blending Research Octane Number (clear) |
| Paraffins: | |
| n-heptane | 0 |
| 2-methylhexane | 41 |
| 3-methylhexane | 56 |
| 2,2-dimethylpentane | 89 |
| 2,3-dimethylpentane | 87 |
| 2,2,3-trimethylbutane | 113 |

### Feedstock

The feedstock for the process is preferably one which contains significant amounts of C₅+ normal and/or slightly branched paraffins, especially normal and/or slightly branched paraffins in the C₆-C₁₀ range. Accordingly, normal hexane and normal heptane as well as the various mono-methyl branched isomers alone or in admixture may be employed as the feedstock in the process of the invention. In addition, the feedstock may contain monocyclic aromatic compounds and/or cyclic paraffins, such as cyclohexane.

The feedstock to the isomerization process can be straight-run, thermal, or catalytically cracked naphtha. Preferably, for high increases in octane numbers of the feed, the charge to the process is a naphtha rich in C₆ to C₁₀ paraffins. Naphtha rich in C₆ and C₇ paraffins is generally difficult to reform selectively using conventional catalysts (such as chlorided Pt-alumina). Naphthas can be obtained by separating the charge into two fractions: a light naphtha and a heavy naphtha. Conventionally such separation is by distillation. The boiling range of the light naphtha is from 27 to 204°C (80 to 400°F) and the boiling range of the heavy naphtha will be up to 343°C (650°F). The light naphtha will be rich in C₆-C₁₀ paraffins, and specifically C₆ and C₇ paraffins. In accordance with one embodiment when the light naphtha is upgraded in accordance with the invention, the heavy naphtha will be processed by conventional reforming.

Generally, the feeds of the process of the invention do not contain bicyclic and polycyclic aromatics; bicyclic and polycyclic aromatics are commonly found in the higher boiling fractions (Initial boiling point over 340°C.) than those used as feeds in the process of the invention . Single ring (monocyclic) aromatics which are readily hydrogenated over the metal component of the catalyst can be tolerated and at the higher end of the range of temperature conditions of the process of the invention may be subject to ring opening to form branched chain paraffin compounds. The aromatic content is preferably held below 10 weight percent although slightly greater amounts up to about 20 weight percent might be tolerated if the proportion of monocyclic aromatics is sufficiently high and if a sufficiently strong hydrogenation component such as platinum is present on the catalyst.

In another specific embodiment of isomerizing lower molecular weight hydrocarbons, the feedstock is a refinery stream which contains significant amounts of C₄-C₆ n-paraffins.

### Catalyst

The catalyst composition employed in the process of the the invention comprises a zeolite having, in its calcined form, an X-ray diffraction pattern including the lines listed in Table a below:

**TABLE A**

| Interplanar d-Spacing (Å) | Relative Intensity, I/Iₒ x 100 |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

or more specifically the lines listed in Table B below:

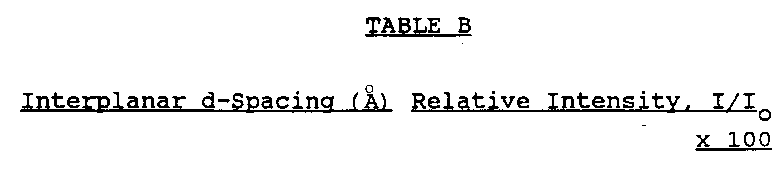

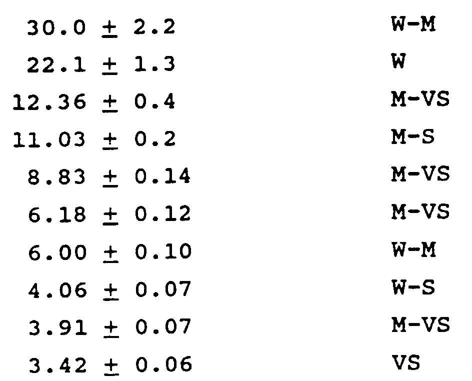

or yet more specifically the lines listed in Table C below:

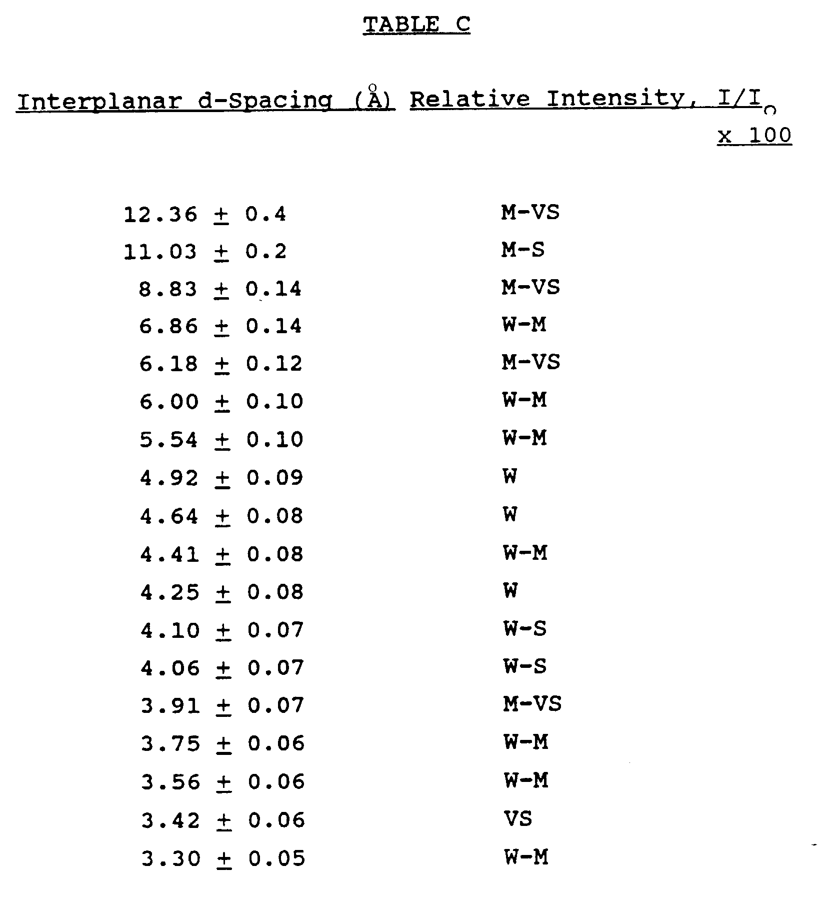

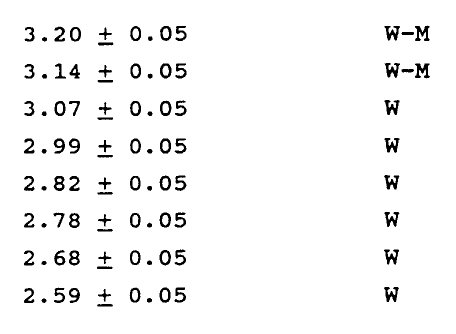

and most preferably the lines listed in Table D below:

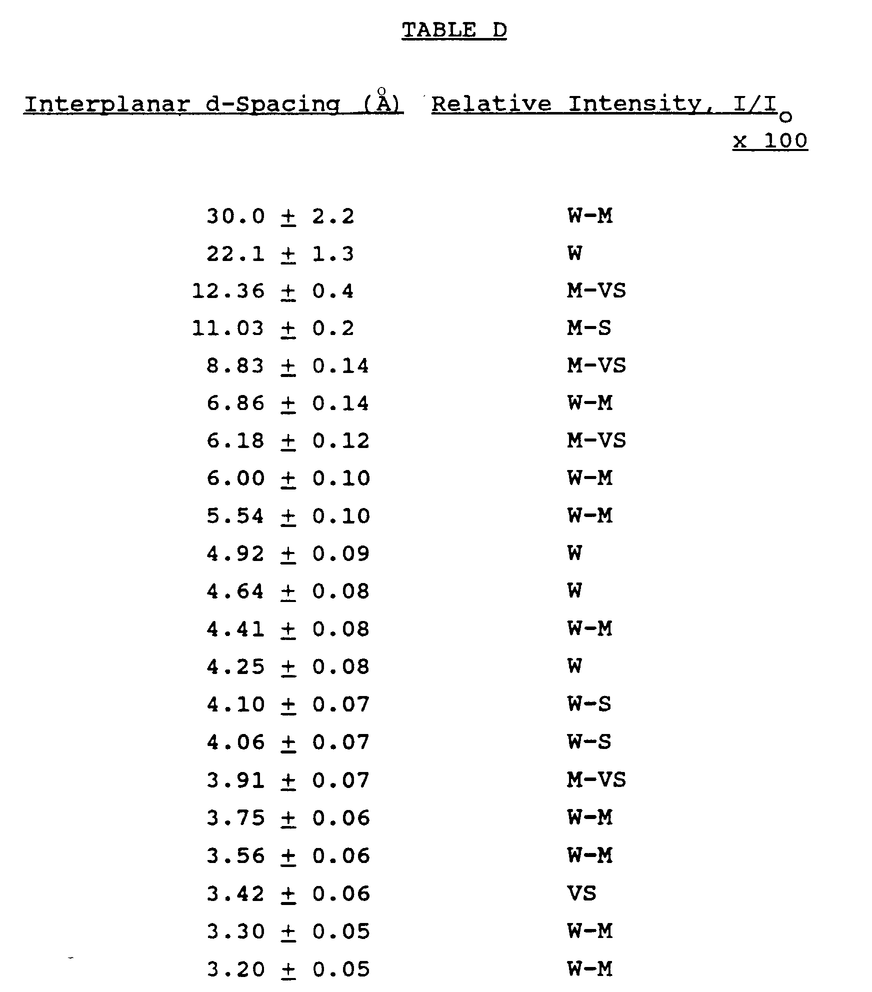

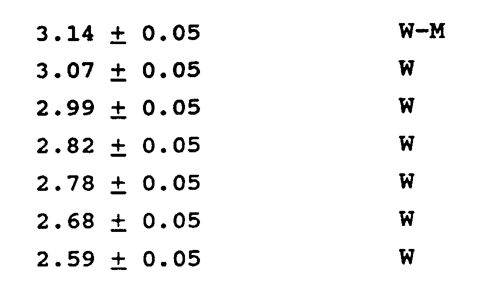

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the diffractometer. From these, the relative intensites, 100 I/Iₒ, where Iₒ is the intensity of the strongest line or peak, and d (obs.) the interplanar spacing in Ångstrom Units (Å), corresponding to the recorded lines, were determined. In Tables A-D, the relative intensities are given in terms of the symbols W = weak, M = medium, S = strong, VS = very strong. In terms of intensities, these may be generally designated as follows:

| | |
|---|---|
| W = | 0-20 |
| M = | 20-40 |
| S = | 40-60 |
| VS = | 60-100 |

Examples of suitable zeolite are the PSH-3 composition of U.S. Patent No. 4,439,409 and MCM-22 as described in International Patent Publication No. WO 90/06283 published June 14, 1990.

Zeolite MCM-22 has a composition involving the molar relationship:

X₂O₃:(n)YO₂,

wherein X is a trivalent element, such as aluminum, boron, iron and/or gallium, preferably aluminum, Y is a tetravalent element such as silicon and/or germanium, preferably silicon, and n is at least 10, usually from 10 to 150, more usually from 10 to 60, and even more usually from 20 to 40. In the as-synthesized form, zeolite MCM-22 has a formula, on an anhydrous basis and in terms of moles of oxides per n moles of YO₂, as follows:

(0.005-0.1)Na₂O:(1-4)R:X₂O₃:nYO₂

wherein R is an organic component, preferably hexamethyleneimine. The Na and R components are associated with the zeolite as a result of their presence during crystallization, and are easily removed by conventional techniques.

Zeolite MCM-22 is thermally stable and exhibits a high surface area greater than about 400 m²/gm as measured by the BET (Bruenauer, Emmet and Teller) test. As is evident from the above formula, MCM-22 is synthesized nearly free of Na cations and thus possesses acid catalytic activity as synthesized. It can, therefore, be used as a component of the catalyst composition herein without having to first undergo an exchange step. To the extent desired, however, the original sodium cations of the as-synthesized material can be replaced in accordance with techniques well known in the art, at least in part, by ion exchange with other cations. Preferred replacement cations include metal ions, hydrogen ions, hydrogen precursor, e.g., ammonium, ions and mixtures thereof. Particularly preferred cations are those which tailor the activity of the catalyst for isomerization. These may include hydrogen, rare earth metals and metals of Groups IIA, IIIA, IVA, IB, IIB, IIIB, IVB and VIII of the Periodic Table of the Elements.

In its calcined form, zeolite MCM-22 appears to be made up of a single crystal phase with little or no detectable impurity crystal phases and has an X-ray diffraction pattern including the lines listed in above Tables A-D.

Zeolite MCM-22 can be prepared from a reaction mixture containing sources of alkali or alkaline earth metal (M), e.g., sodium or potassium, cation, an oxide of trivalent element X, e.g, aluminum, an oxide of tetravalent element Y, e.g., silicon, an organic (R) directing agent, preferably hexamethyleneimine, and water, said reaction mixture having a composition, in terms of mole ratios of oxides, within the following ranges:

| Reactants | Useful | Preferred |
|---|---|---|
| YO₂/X₂O₃ | 10 - 60 | 10 - 40 |
| H₂O/YO₂ | 5 - 100 | 10 - 50 |
| OH⁻/YO₂ | 0.01 - 1.0 | 0.1 - 0.5 |
| M/YO₂ | 0.01 - 2.0 | 0.1 - 1.0 |
| R/YO₂ | 0.05 - 1.0 | 0.1 - 0.5 |

In a preferred method of synthesizing zeolite MCM-22, the YO₂ reactant contains a substantial amount of solid YO₂, e.g., at least about 30 wt.% solid YO₂. Where YO₂ is silica, the use of a silica source containing at least about 30 wt.% solid silica, e.g., Ultrasil (a precipitated, spray dried silica containing about 90 wt.% silica) or HiSil (a precipitated hydrated SiO₂ containing about 87 wt.% silica, about 6 wt.% free H₂O and about 4.5 wt.% bound H₂O of hydration and having a particle size of about 0.02 micron) favors crystal formation from the above mixture. If another source of oxide of silicon, e.g., Q-Brand (a sodium silicate comprised of about 28.8 wt.% of SiO₂, 8.9 wt.% Na₂O and 62.3 wt.% H₂O) is used, crystallization may yield little if any MCM-22 crystalline material and impurity phases of other crystal structures, e.g., ZSM-12, may be produced. Preferably, therefore, the YO₂, e.g., silica, source contains at least about 30 wt.% solid YO₂, e.g., silica, and more preferably at least about 40 wt.% solid YO₂, e.g., silica.

Crystallization of the MCM-22 crystalline material can be carried out at either static or stirred conditions in a suitable reactor vessel such as, e.g., polypropylene jars or TEFLON®-lined or stainless steel autoclaves at a temperature of 80°C to 225°C for a time of 25 hours to about 60 days. Thereafter, the crystals are separated from the liquid and recovered.

Synthesis of the MCM-22 crystals is facilitated by the presence of at least 0.01 percent, preferably 0.10 percent and still more preferably 1 percent, seed crystals (based on total weight) of the crystalline product.

The catalyst employed in the process of the invention also includes tungsten, molybdenum, rhenium, nickel, cobalt, platinum or palladium. Such component can be introduced in the catalyst composition by way of cocrystallization, exchanged into the composition to the extent a Group IIIA element, e.g., aluminum, is in the zeolite structure, impregnated therein or intimately physically admixed therewith. Such component can be impregnated in, or on, the zeolite such as, for example, by, in the case of platinum, treating the zeolite with a solution containing a platinum metal-containing ion. Thus, suitable platinum compounds for this purpose include chloroplatinic acid, platinous chloride and various compounds containing the platinum amine complex. The amount of the dehydrogenation/hydrogenation component in the catalyst composition preferably ranges from 0.01 to 20 weight percent of the composition.

In addition, it may be desirable to incorporate the zeolite catalyst with another material which is resistant to the temperatures and other conditions employed in the process of this invention. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with the zeolite, i.e., combined therewith or present during its synthesis, which itself is catalytically active may change the conversion and/or selectivity of the catalyst. Inactive materials suitably serve as diluents to control the amount of conversion so that products can be obtained economically and orderly without employing other means for controlling the rate of reaction. These materials may be incorporated into naturally occurring clays, e.g., bentonite and kaolin, to improve the crush strength of the catalyst under commercial operating conditions. Said materials, i.e., clays, oxides, etc., function as binders for the catalyst. It is desirable to provide a catalyst having good crush strength because in commercial use, it is desirable to prevent the catalyst from breaking down into powder-like materials. These clay binders have been employed normally only for the purpose of improving the crush strength of the catalyst.

Naturally occurring clays which can be composited with zeolite crystals include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the zeolite also include inorganic oxides, notably alumina.

In addition to the foregoing materials, the crystals can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia silica-alumina-magnesia and silica-magnesia-zirconia. It may also be advantageous to provide at least a part of the foregoing matrix materials in colloidal form so as to facilitate extrusion of the bound catalyst component(s).

The relative proportions of finely divided crystalline material and inorganic oxide matrix vary widely, with the crystal content ranging from 1 to 90, preferably 2 to 80, weight percent of the composite.

The stability of the catalyst of the invention may be increased by steaming. Suitable steam stabilization conditions include contacting the catalyst with 5-100% steam at a temperature of at least 300°C (e.g., 300-650°C) for at least one hour (e.g., 1-200 hours) at a pressure of 100-2,500 kPa. In a more particular embodiment, the catalyst can be made to undergo steaming with 75-100% steam at 315°-500°C and atmospheric pressure for 2-25 hours.

### Process conditions

The feedstock is contacted with the catalyst composition of the invention in the presence or absence of added hydrogen at a temperature of 120 to 370°C (250 to 700°F), preferably 200 to 315°C (400 to 600°F). Because cracking reactions tend to increase with increasing temperatures, lower temperatures will normally be preferred in order to favor the isomerization over the cracking reactions. Pressures range from 100 to 7000 kPa (atmospheric to 1000 psig), preferably from 450 to 3550 kPa (50 to 500 psig). Weight hourly space velocity is generally from 0.1 to 50 hr⁻¹, more usually 0.2 to 10hr⁻¹. If hydrogen is cofed with the feedstock, the hydrogen:feedstock molar ratio is generally from 1:1 to 10:1.

The process is preferably carried out in the presence of hydrogen, both to inhibit catalyst aging and to promote the isomerization reactions which are thought to proceed through an unsaturated intermediate.

The conversion may be conducted by contacting the feedstock with a fixed stationary bed of catalyst, a fixed fluidized bed or with a transport bed. A simple configuration is a trickle-bed operation in which the feed is allowed to trickle through a stationary fixed bed. With such a configuration, it is desirable to initiate the reaction with fresh catalyst at a moderate temperature which is raised if the catalyst ages, in order to maintain catalytic activity. The catalyst may be regenerated by contact at elevated temperature with hydrogen gas, for example, or by burning in air or other oxygen-containing gas.

The invention will now be more particularly described with reference to the following examples and the accompanying drawing, which is a graph of isoparaffin production vs. temperature for the process of Example 2.

In the examples, whenever sorption data are set forth for comparison of sorptive capacities for water, cyclohexane and/or n-hexane, they were Equilibrium Adsorption values determined as follows:

A weighed sample of the calcined adsorbent was contacted with the desired pure adsorbate vapor in an adsorption chamber, evacuated to less than 1 mm Hg and contacted with 1.6 kPa (12 Torr) of water vapor or 5.3 kPa (40 Torr) of n-hexane or 5.3 kPa (40 Torr) cyclohexane vapor, pressures less than the vapor-liquid equilibrium pressure of the respective adsorbate at 90°C. The pressure was kept constant (within about ± 0.5 mm Hg) by addition of adsorbate vapor controlled by a manostat during the adsorption period, which did not exceed about 8 hours. As adsorbate was adsorbed by the crystalline material, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant.

### EXAMPLE 1

MCM-22 was prepared by dissolving one part sodium aluminate (43.5% Al₂O₃, 32.2% Na₂O, 25.6% H₂O) in a solution containing 1 part of 50% NaOH solution and 103.13 parts H₂O. To this was added 4.50 parts hexamethyleneimine. The resulting solution was added to 8.55 parts of Ultrasil, a precipitated, spray-dried silica (90% SiO₂).

The reaction mixture had the following composition, in mole ratios:

| | |
|---|---|
| SiO₂/Al₂O₃ | = 30.0 |
| OH/SiO₂ | = 0.18 |
| H₂O/SiO₂ | = 44.9 |
| Na/SiO₂ | = 0.18 |
| R/SiO₂ | = 0.35 |

where R is hexamethyleneimine.

The mixture was crystallized in a stainless steel reactor, with stirring, at 150°C for 7 days. The crystalline product was filtered, washed with water, dried at 120°C and calcined for 20 hours at 538°C. The sorption capacities of the calcined material were measured to be:

| | |
|---|---|
| H₂O | 15.2 wt% |
| Cyclohexane | 14.6 wt% |
| n-Hexane | 16.7 wt% |

The surface area of the zeolite was measured to be 494 m²/g.

To produce the required isomerization catalyst, the resultant MCM-22 was mixed with an alumina binder and the mixture was mulled, extruded and dried at 120°C (250°F). It was then calcined in nitrogen at 540°C (1000°F) for 3 hours. After humidification the mixture was exchanged with 1N ammonium nitrate, dried and calcined again in nitrogen at 540°C (1000°F). The mixture was again humidified, exchanged with Pt(NH₃)₄Cl₂ solution for 8 hours, rinsed, dried and calcined at 350°C (660°F) in air. Catalyst properties are given in Table 1.

**TABLE 1**

| PT/MCM-22 CATALYST PROPERTIES | |
|---|---|
| Composition, wt% | |
| Zeolite MCM-22 | 65 |
| Platinum | 0.66 |
| Binder | remainder |

| Density, g/cc | |
|---|---|
| Packed | 0.45 |
| Particle | 0.73 |
| Real | 2.60 |

| Physical Properties | |
|---|---|
| Pore Volume, cc/g | 0.99 |
| Surface Area, m²/g | 372 |
| Avg. Pore Diameter, Å | 106 |

### EXAMPLE 2

The activity of the Pt/MCM-22 catalyst produced in Example 1 was compared against a Pt/silica-alumina catalyst (0.6 wt% Pt) and UOP's I-7, a commercially available noble metal containing mordenite-based isomerization catalyst, using a microunit equipped with a 1.3 cm (1/2") downflow stainless steel reactor. In a typical experiment, 10 cm³ of sized catalyst (14/24 mesh) were loaded into the reactor and reduced in hydrogen at 427°C (800°F) for 2 hours. The reactor was cooled to 232°C (450°F) and pressurized to 790kPa (100 psig) with hydrogen. A n-hexane feed was then introduced at 1 gram/gram cat/hr with a 2/1 mol/mol hydrogen co-feed. The product from the reactor was analyzed with an on-line gas chromatograph equipped with a 30 meter megabore DB-1 column.

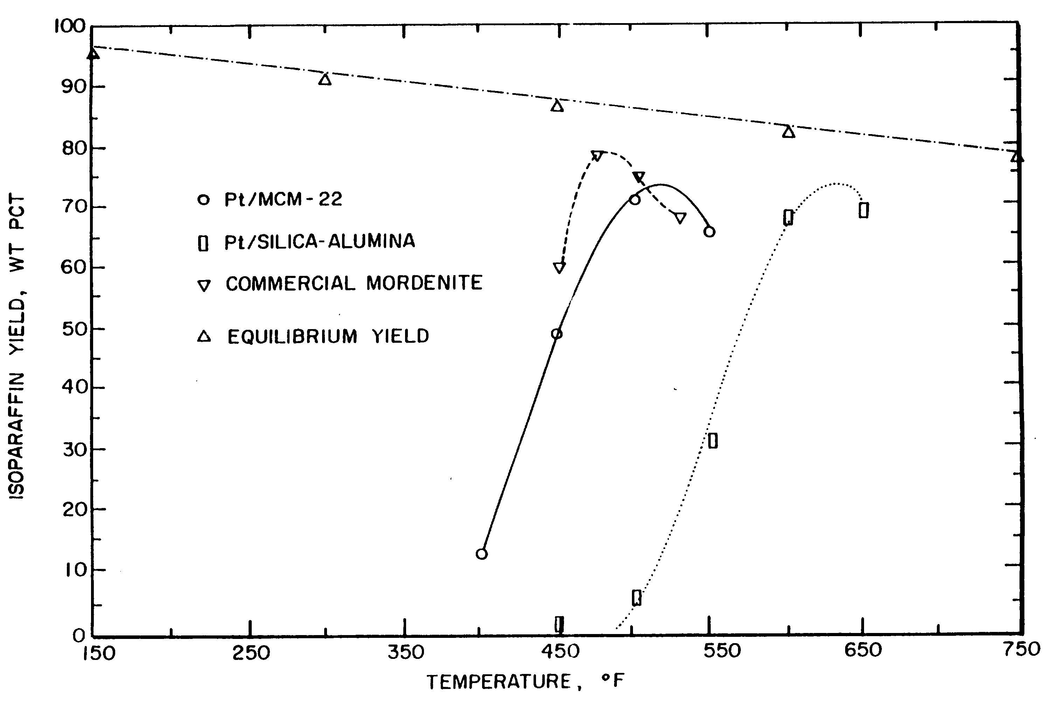

The yields of isoparaffin products are plotted as a function of temperature in Figure 1. The Pt/MCM-22 and I-7 catalysts showed comparable activity and afforded near equilibrium conversion of n-hexane at 246-260°C (475-500°F) while the Pt/silica-alumina catalyst required a much higher temperature, 330°C (625°F), for near equilibrium conversions of n-hexane.

## Claims

1. A process for isomerizing a paraffin having 4 to 10 carbon atoms comprising contacting the paraffin with a catalyst composition comprising:
(i) tungsten, molybdenum, rhenium, nickel, cobalt, platinum, or palladium; and
(ii) a zeolite which exhibits an x-ray diffraction pattern including the following lines:
| Interplanar d-Spacing (Å) | Relative Intensity, I/Iₒ x 100 |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

2. A process as claimed in claim 1 wherein the zeolite exhibits an x-ray diffraction pattern including the following lines:
| Interplanar d-Spacing (Å) | Relative Intensity, I/Iₒ x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

3. A process as claimed in Claim 1 wherein the zeolite exhibits an x-ray diffraction pattern including the following lines:
| Interplanar d-Spacing (Å) | Relative Intensity, I/Iₒ x 100 |
|---|---|
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

4. A process as claimed in Claim 1 wherein the zeolite exhibits an x-ray diffraction pattern including the following lines:
| Interplanar d-Spacing (Å) | Relative Intensity, I/Iₒ x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

5. A process as claimed in any preceding claim wherein said contacting is effected under conditions comprising a temperature 120 to 370°C (250 to 700°F), a pressure of 100 to 7000 kPa (atmospheric to 1000 psig), a weight hourly space velocity of 0.1 to 50 hr⁻¹, and a hydrogen:feedstock molar ratio of 0 (no added hydrogen) to 10:1.

6. A process as claimed in Claim 5, wherein the temperature is 200 to 315°C (400 to 600°F).

7. A process as claimed in Claim 5, wherein the pressure is 450 to 3550 kPa (50 to 500 psig).

8. A process as claimed in Claim 5, wherein the weight hourly space velocity is 0.2 to 10 hr⁻¹.

9. A process as claimed in Claim 5, wherein the hydrogen:feedstock molar ratio is 1:1 to 10:1.

10. A process as claimed in any preceding claim, wherein the catalyst composition contains 0.01 to 20% of weight of the dehydrogenation/hydrogenation metal.

## Patentansprüche

1. Verfahren zur Isomerisierung eines Paraffins mit 4 bis 10 Kohlenstoffatomen, bei dem man das Paraffin mit einer Katalysatorzusammensetzung, enthaltend:
(i) Wolfram, Molybdän, Rhenium, Nickel, Cobalt, Platin oder Palladium und
(ii) einen Zeolithen, dessen Röntgenbeugungsmuster die folgenden Linien enthält:
| Interplanarer d-Abstand (Å) | Relative Intensität, I/Iₒx100 |
|---|---|
| 12,36 ± 0,4 | M-SS |
| 11,03 ± 0,2 | M-S |
| 8,83 ± 0,14 | M-SS |
| 6,18 ± 0,12 | M-SS |
| 6,00 ± 0,10 | W-M |
| 4,06 ± 0,07 | W-S |
| 3,91 ± 0,07 | M-SS |
| 3,42 ± 0,06 | SS |
in Berührung bringt.

2. Verfahren nach Anspruch 1, bei dem man einen Zeolithen einsetzt, dessen Röntgenbeugungsmuster die folgenden Linien enthält:

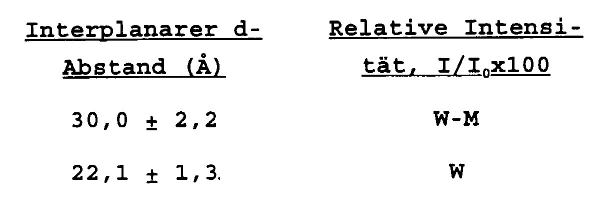

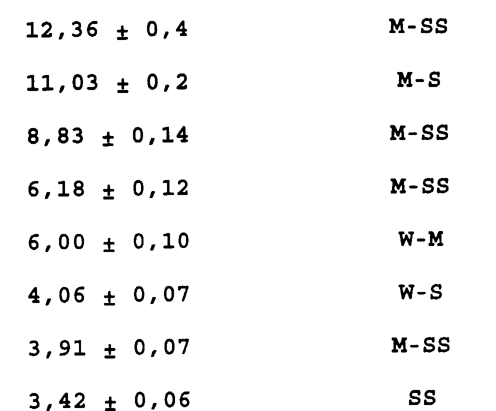

3. Verfahren nach Anspruch 1, bei dem man einen Zeolithen einsetzt, dessen Röntgenbeugungsmuster die folgenden Linien enthält:

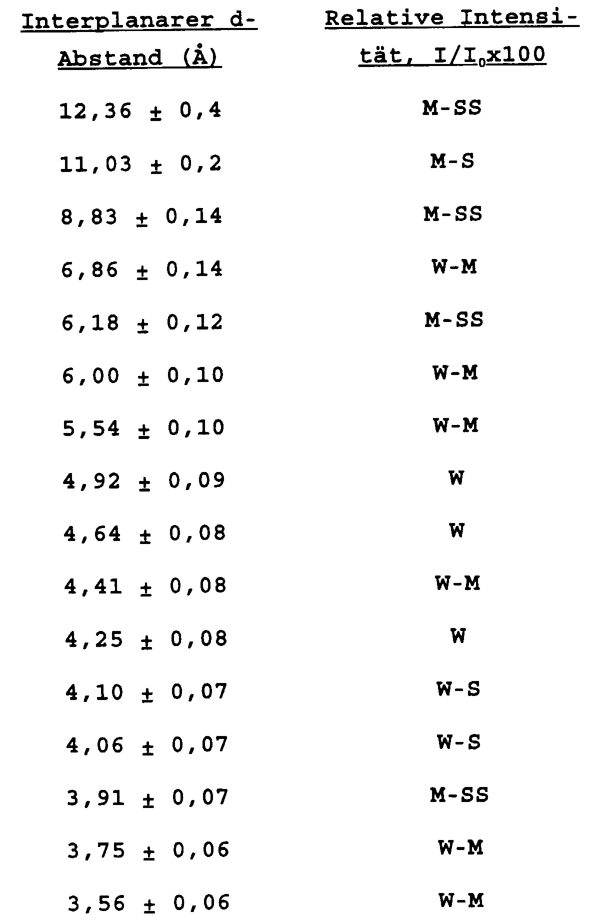

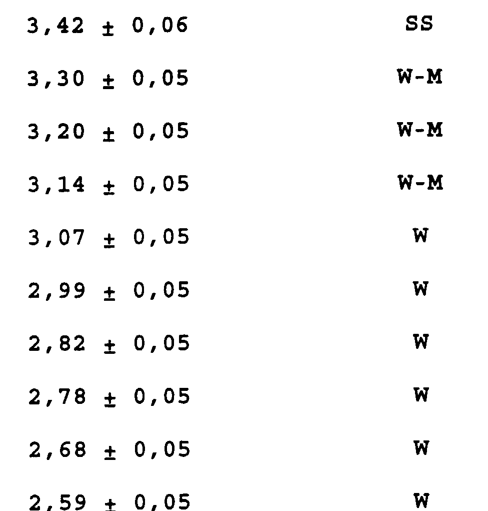

4. Verfahren nach Anspruch 1, bei dem man einen Zeolithen einsetzt, dessen Röntgenbeugungsmuster die folgenden Linien enthält:

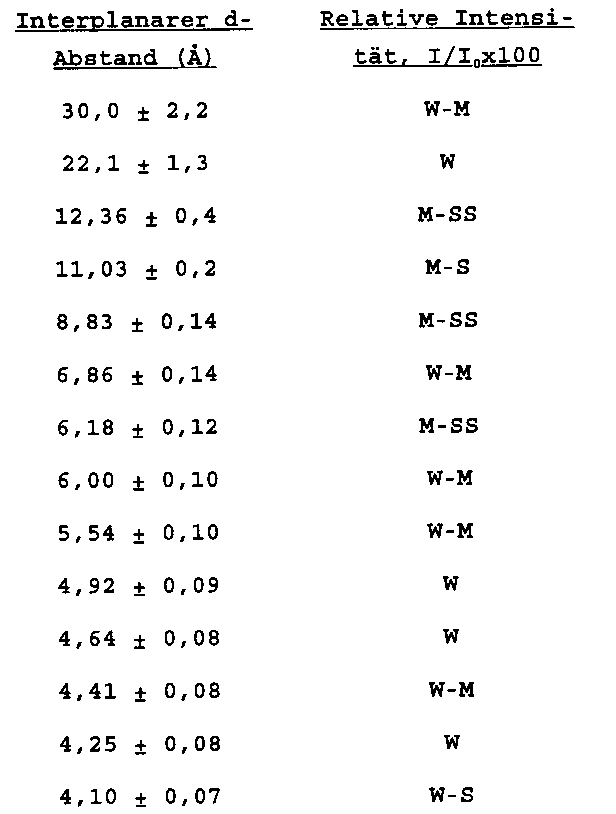

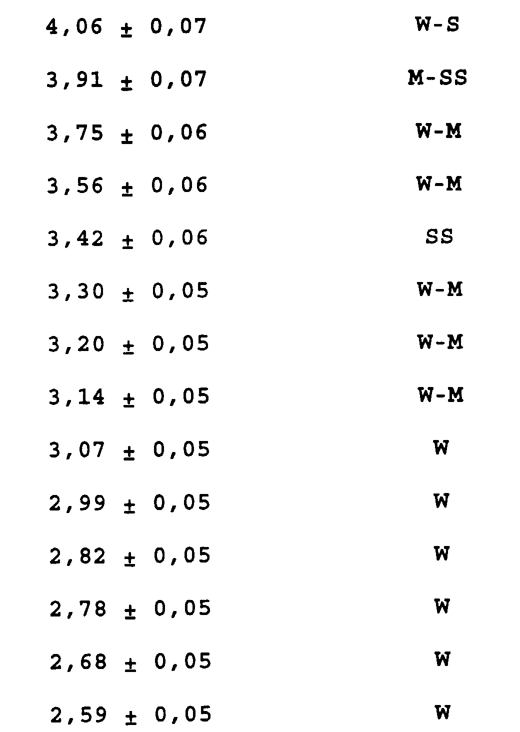

5. Verfahren nach einem der vorhergehenden Ansprüche, bei dem man das Inberührungbringen bei einer Temperatur von 120 bis 370°C (250 bis 700°F), einem Druck von 100 bis 7000 kPa (Normaldruck bis 1000 psi Überdruck), einer Katalysatorbelastung von 0,1 bis 50 h⁻¹ und einem Molverhältnis von Wasserstoff zu Einsatzstoff von 0 (kein Wasserstoffzusatz) bis 10:1 durchführt.

6. Verfahren nach Anspruch 5, das man bei einer Temperatur von 200 bis 315°C (400 bis 600°F) durchführt.

7. Verfahren nach Anspruch 5, das man bei einem Druck von 450 bis 3550 kPa (50 bis 500 psi Überdruck) durchführt.

8. Verfahren nach Anspruch 5, das man bei einer Katalysatorbelastung von 0,2 bis 10 h⁻¹ durchführt.

9. Verfahren nach Anspruch 5, das man bei einem Molverhältnis von Wasserstoff zu Einsatzstoff von 1:1 bis 10:1 durchführt.

10. Verfahren nach einem der vorhergehenden Ansprüche, bei dem man eine Katalysatorzusammensetzung einsetzt, die 0,01 bis 20 Gew.-% des Dehydrierungs-Hydrierungs-Metalls enthält.

## Revendications

1. Procédé d'isomérisation d'une paraffine possédant de 4 à 10 atomes de carbone, caractérisé en ce que l'on met la paraffine en contact avec une composition catalytique comprenant:
(i) du tungstène, du molybdène, du rhénium, du nickel, du cobalt, du platine, ou du palladium; et
(ii) une zéolite qui présente un réseau de diffraction des rayons X comprenant les raies suivantes:
| Espacement-d interplanaire (Å) | Intensité relative, I/Iₒ x 100 |
|---|---|
| 12,36±0,4 | M-TP |
| 11,03±0,2 | M-P |
| 8,83±0,14 | M-TP |
| 6,18±0,12 | M-TP |
| 6,00±0,10 | F-M |
| 4,06±0,07 | F-P |
| 3,91±0,07 | M-TP |
| 3,42±0,06 | TP |

2. Procédé suivant la revendication 1, caractérisé en ce que la zéolite présente un réseau de diffraction des rayons X comprenant les raies suivantes:
| Espacement-d interplanaire (Å) | Intensité relative, I/Iₒ x 100 |
|---|---|
| 30,0±2,2 | F-M |
| 22,1±1,3 | F |
| 12,36±0,4 | M-TP |
| 11,03±0,2 | M-P |
| 8,83±0,14 | M-TP |
| 6,18±0,12 | M-TP |
| 6,00±0,10 | F-M |
| 4,06±0,07 | F-P |
| 3,91±0,07 | M-TP |
| 3,42±0,06 | TP |

3. Procédé suivant la revendication 1, caractérisé en ce que la zéolite présente un réseau de diffraction des rayons X comprenant les raies suivantes:

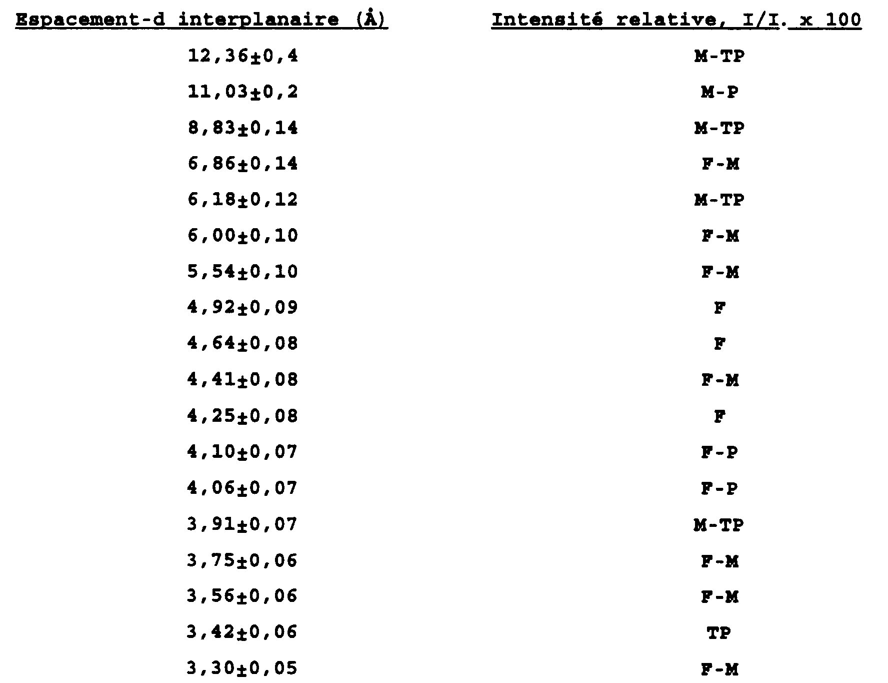

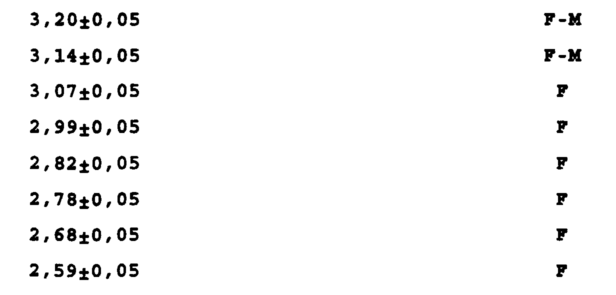

4. Procédé suivant la revendication 1, caractérisé en ce que la zéolite présente un réseau de diffraction des rayons X comprenant les raies suivantes:

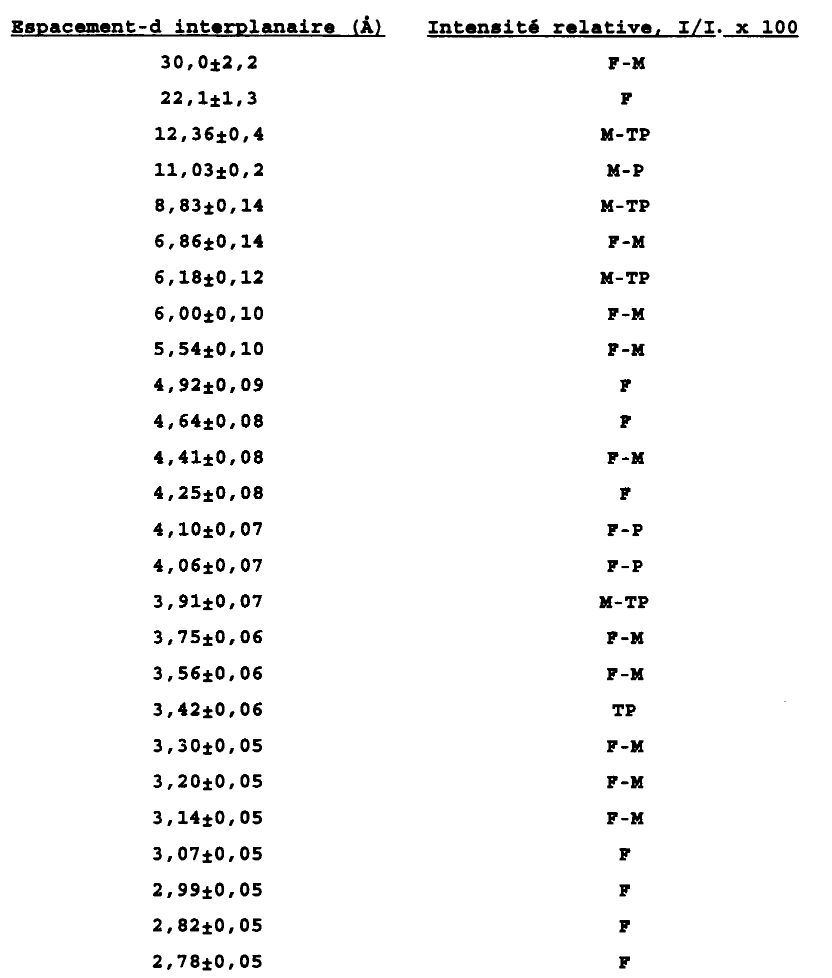

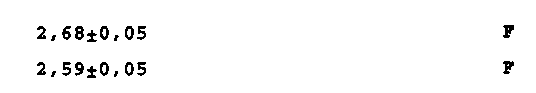

5. Procédé suivant l'une quelconque des revendications précédentes, caractérisé en ce que l'on procède à la mise en contact dans des conditions comprenant une température de 120 à 370°C (250 à 700°F), une pression de 100 à 7 000 kPa (pression atmosphérique à 1000 psig), une vitesse spatiale horaire pondérale de 0,1 à 50 h⁻¹ et un rapport molaire hydrogène:charge de départ de 0 (pas d'hydrogène ajouté) à 10:1.

6. Procédé suivant la revendication 5, caractérisé en ce que la température varie de 200 à 315°C (400 à 600°F).

7. Procédé suivant la revendication 5, caractérisé en ce que la pression varie de 450 à 3 550 kPa (50 à 500 psig).

8. Procédé suivant la revendication 5, caractérisé en ce que la vitesse spatiale horaire pondérale est de 0,2 à 10 h⁻¹.

9. Procédé suivant la revendication 5, caractérisé en ce que le rapport molaire hydrogène:charge de départ varie de 1:1 à 10:1.

10. Procédé suivant l'une quelconque des revendications précédentes, caractérisé en ce que la composition catalytique contient de 0,01 à 20% en poids du métal de déshydrogénation/hydrogénation.
